# EUROPEAN PATENT APPLICATION

(11) **EP 4 215 224 A1**
(43) Date of publication of application: **26.07.2023**
(21) Application number: 22153259.1
(22) Date of filing: 25.01.2022
(51) Int. Cl.: A61L 27/18, A61L 27/34, A61L 27/54, A61L 31/06, A61L 31/10, A61L 31/16

(54) **TISSUE PROTECTION**

(71) Applicant: BIOTRONIK AG, 8180 Bülach (CH)
(72) Inventor: Quint, Bodo, 79802 Dettighofen (DE)
(74) Representative: Biotronik Corporate Services SE

(57) **Abstract**

The present invention relates to a medical device (1) comprising a polymer, the polymer being an α-ketoglutaric acid-based polymer.

## Description

The present invention relates to a medical device and a use of an α-Ketoglutaric acid-based polymer.

Progressive cell injury is triggered by cell death of individual tissue cells by a mechanism described as "reperfusion injury". However, such damage also occurs when areas of tissue are restored to unrestricted blood flow after severely depleted blood flow. Primarily, surgical methods attempt to minimize this damage by pharmacological support, but pharmacological therapy of acute myocardial infarction is also increasingly addressing this type of cellular damage.

In the prior art it is known to pharmacologically counteract tissue damage by oxidative (inflammatory) effects.

While percutaneous coronary intervention (PCI) has become the much more promising treatment method during acute cardiac care than pharmaceutically induced lysis of vascular blockade, the success of PCI has long been based not merely on the advancement of the catheter instruments themselves, but to a higher degree on the sophisticated use of pharmaceutical adjuvants. This applies not only to lysis therapy, which has evolved to accompany the use of primary percutaneous coronary intervention (pPCI), but also to pharmaceutical cardio protection. In the meantime, PCI has been optimized to a large extent and is proving to be reproducible enough to allow effects of reperfusion therapy to become detectable already in clinical trials. Originally suspected biological correlations from biological cell research are now also being investigated in the setting of PCI. One systematic known long term pathway of the inflammatory respiration injury is caused by "oxidative stress" which harms primary cell membranes. Interestingly, the main cell damage - attributed by literature - starts almost immediately with the mechanical harm of vessel tissue (bleeding into the tissue, for example caused by tissue dilation) or the reperfusion process itself indirectly triggered by already existing cell damage. Especially in AMI cases (also called pPCI if treated by PCI) a significant amount of acute cell damage is expected within the reperfusion path following a vessel striction.

Reperfusion catheters are known from the prior art and can perform a tissue-protective function, but are only applicable for a short time. In addition, during a pPCI, too intensive catheter interventions are systematically avoided because of the risk of distal embolism if the catheter intervention is too intensive. Meanwhile, a pPCI follows the strategy to provide a reperfusion of the heart as fast as possible and also as comprehensive as possible, but generally more complex interventions are still avoided in a first catheter intervention.

Balloon angioplasty, in particular the stenting of vascular occlusions, has emerged as a potent technology to treat undersupply of areas of the body due to blood vessel occlusion. The effects of reperfusion injury have been scientifically known for decades, but this knowledge has primarily been applied to surgical procedures. The success of PCI, however, is not exclusively based on the medical-technical advancement of the catheter products, but these PCI procedures are also perfected by an ever more sophisticated supporting pharmacological therapy. So far, PCI has continued to evolve with respect to the most pressing problem "restenosis". Interestingly, after the establishment of the drug eluting stent (DES), clinical trials of the so-called pPCI cases (initial treatment of acute myocardial infarction) have already resulted in instructions that consider reperfusion tissue damage of the pPCI. Based on clinical experience, since 2020, when the ECH Guideline 2019 has come into force, the previously established intensive oxygen supply of patients with acute myocardial infarction is almost dispensed with. Only when the relative oxygen content of the patient's arterial blood is < 90% should the patient now receive oxygen (amendment to the ECH Guideline of 2017). This is based on the clinical evidence that a limitation of oxidative tissue damage after reperfusion by a reduced blood oxygen content in the subsequent disease course of pPCI are to be noticed.

AMI (acute myocardial infarction) cases represent an ever-increasing proportion of coronary catheter intervention (between 30-50% to date) - and this proportion will continue to rise to over 50% of PCI cases in the future as catheter interventions become more successful. The drug eluting stent can so far limit the re-occlusion of a treated stenosis - what if there would be the possibility to additionally influence the healing process of the affected tissue following the stenosis in a sustainable way? In this respect, such a measure had to be particularly valuable also for PTA (percutaneous transluminal angioplasty) cases.

Based on the above, there is a fundamental need for an efficient release of a cardio- or tissue-protective agent, particularly after an intense vascular occlusion.

This problem is solved by a medical device having the features of claim 1 and a use having the features of claim 14.

Preferred embodiments of these aspects of the present invention are stated in the corresponding dependent claims and are described below.

According to claim 1 a medical device comprising an α-ketoglutaric acid-based polymer is disclosed, wherein particularly α-ketoglutaric acid comprises the chemical formula C₅H₆O₅ (CAS Number 328-50-7) and the structural formula:

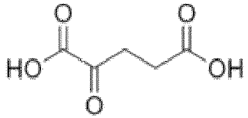

Particularly, according to an embodiment, the α-ketoglutaric acid-based polymer is adapted to be hydrolysable such that, when hydrolyzed, releases at least one cardio- or tissue-protective substance.

Particularly, in an embodiment, said α-ketoglutaric acid-based polymer may also serve as a polymer matrix for the protective agents themselves, of which the polymer is composed, to provide short-term intensified release kinetics following tissue dilatation at the site of implementation and for the affected blood supply area. It is found that such a polymer matrix can also be used synergistically with an anti-proliferative agent, since this agent has at least antioxidant, but also inflammation-reducing activity.

Advantageously, α-ketoglutaric acid acts both chemically as a reducing agent towards oxidative species, but also metabolically very effectively against this reperfusion injury. The oxygen uptake capacity of tissue cells is increased by the metabolic availability of this substance.

α-ketoglutaric acid represents a multifunctional molecule - it is suitable for the construction of a macromolecular compound (Robert Taschner (2019), Diploma Thesis, Institute of Applied Synthetic Chemistry, Technical University Vienna: "Biocompatible Photoinitiators based on α-ketoesters). Within polymer chemistry it is often recognized that monomers are soluble within the polymer matrix. Since in the above mentioned case polymers and monomers are considered polar, it can be assumed that this monomer is soluble in the polymer matrix. Particularly, since this compound is considered a stronger reducing agent than ascorbic acid, it must also be excellent at providing chemical stabilization for substances such as sirolimus / rapamycin. In an embodiment, the α-ketoglutaric acid-based polymer is therefore also used as a polymer matrix of a drug eluting stent according to an embodiment, see further below. With advantage the hydrolysis products are beneficial by counteracting inflammation through releasing α-ketoglutaric acid during hydrolysis of the polymer.

According to a preferred embodiment of the present invention, the α-ketoglutaric acid-based polymer is an α-ketoglutaric acid-based polyester.

Further, in an embodiment, the α-ketoglutaric acid-based polymer is selected from the group comprised of: α-ketoglutaric hexanediol polyester, α-ketoglutaric isosorbide polyester,

Particularly, α-ketoglutaric hexanediol polyester or α-ketoglutaric isosorbide polyester can be obtained according to the following reaction as shown by Taschner (see reference above)

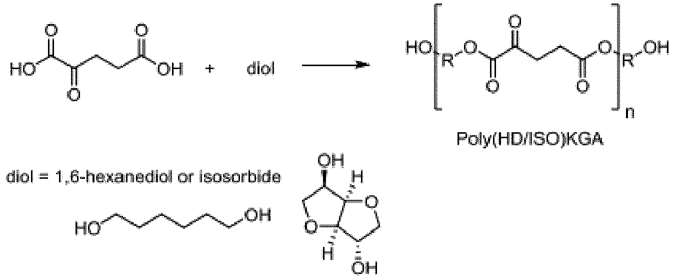

Furthermore, according to a preferred embodiment, the medical device is an implantable medical device.

Further, in an embodiment, the medical device is a stent. According to a preferred embodiment, the stent is a drug eluting stent configured to release a drug.

Further, in an embodiment, the stent comprises a scaffold coated with a polymer matrix, the polymer matrix comprising or being formed by said α-ketoglutaric acid-based polymer (particularly α-ketoglutaric acid-based polyester as disclosed herein), the drug being dispersed in said polymer matrix. Particularly, the polymer matrix binds the drug to the stent and modulate the elution of the drug into the tissue of a person when the stent is in an implanted state (in which it is implanted into the person, particularly into a vessel of the person).

Furthermore, in an embodiment, said α-ketoglutaric acid-based polymer is spun onto the scaffold, particularly using electrospinning.

According to a further embodiment, said drug is preferably an anti-proliferative drug. In a specific embodiment the drug is selected from the group comprised of: sirolimus (C₅₁H₇₉NO₁₃, CAS Number 53123-88-9) or derivatives thereof, colchicine (C₂₂H₂₅NO₆, CAS Number 64-86-8), paclitaxel or combinations of the aforementioned drugs. In a particularly preferred embodiment the drug is colchicine. With colchicine as anti-proliferative drug and the use of an α-ketoglutaric acid-based polymer as described herein a medical device can be provided exhibiting an extremely low potential for inflammation. As described above the hydrolysis products of a α-ketoglutaric acid-based polymer are beneficial by counteracting inflammation through releasing α-ketoglutaric acid. Furthermore, colchicine has shown a significant inhibition of interaction between white blood cells (WBC) and endothelial cells interfering with their transmigration. Thereby, colchicine's anti-inflammatory effect is multifaceted and in combination with an α-ketoglutaric acid-based polymer a specifically, synergetic anti-inflammatory coating can be provided.

According to a further embodiment, the medical device is a graft, particularly a graft for repairing an aneurysm.

Further, in an embodiment, the medical device is a prosthetic heart valve, the heart valve prosthesis comprising a component comprising said α-ketoglutaric acid-based polymer.

Particularly, in an embodiment, said component is one of: a skirt (e.g. arranged on a scaffold of the heart valve prosthesis); a valve member of the heart valve prosthesis, the valve member comprising a plurality of valve leaflets. It is also conceivable to provide an appendage closure device or a septum closure device comprising a cover made of or comprising said α-ketoglutaric acid-based polymer as defined herein.

According to a further embodiment, the medical device is a wound patch comprising a surface, the surface being formed by said α-ketoglutaric acid-based polymer or comprising said α-ketoglutaric acid-based polymer.

Further, in an embodiment, the medical device is a surgical mesh, particularly a hernia mesh (i.e. a surgical mesh configured for hernia repair). The mesh can be formed out the α-ketoglutaric acid-based polymer or can comprise such a polymer. Particularly, a surface of the mesh can be formed by such a polymer or can comprise such a polymer.

According to yet another aspect of the present invention, a use of an α-ketoglutaric acid-based polymer as a material of a medical device is disclosed. According to an embodiment, the material is a surface material of the medical device. Furthermore, in an embodiment, the medical device is an implantable medical device.

Furthermore, according to an embodiment of the use according to the present invention, said α-ketoglutaric acid-based polymer is one of: an α-ketoglutaric acid-based polyester, α-ketoglutaric hexanediol polyester, α-ketoglutaric isosorbide polyester.

In the following, embodiments as well as further features and advantages of the present invention shall be described with reference to the Figures, wherein
- Fig. 1: shows an embodiment of a medical device according to the present invention in form of a drug eluting stent comprising a polymer matrix comprising α-ketoglutaric acid-based polymer;
- Fig. 2: shows an embodiment of a medical device according to the present invention in form of a graft, here as an example for repairing an aneurysm, the graft comprising α-ketoglutaric acid-based polymer;
- Fig. 3: shows an embodiment of a medical device according to the present invention in form of a prosthetic heart valve comprising α-ketoglutaric acid-based polymer;
- Fig. 4: shows an embodiment of a medical device according to the present invention in form of a wound patch comprising α-ketoglutaric acid-based polymer; and
- Fig. 5: shows an embodiment of a medical device according to the present invention in form of a surgical mesh comprising α-ketoglutaric acid-based polymer.

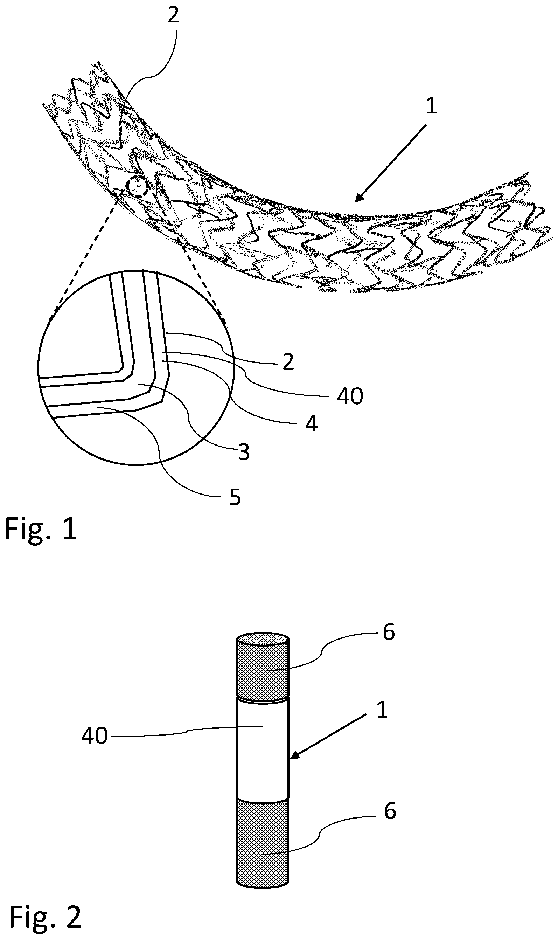

Fig. 1 shows an embodiment of a medical device 1 according to the present invention, wherein an α-ketoglutaric acid-based polymer, particularly according to examples of such polymers disclosed herein, is used as a polymer matrix 4 of a drug eluting stent 1.

When such a stent 1 is placed, an existing undersupply of the subsequent tissue (which can be reached by this perfusion route) can often be assumed. This cellular damage is particularly pronounced when acute stenting (e.g., ST-elevation myocardial infarction, or STEMI) has been identified. Advantageously, the ketoglutaric acid is an agent that results in both biochemical stabilization of the cellular environment and ROS protection. However, in the inflammatory mechanism, the formation of (R)eactive (O)xygen (S)pecies is a rather secondary effect of the consequent inflammation. For this reason, the polymer matrix layer (e.g. in the context of an acute STEMI treatment) can be combined with a direct inhibition of inflammation by an immediate physical release of an agent that directly inhibits the immigration of neutrophils into the tissue and their "diversification". This would then be mechanistically at the very beginning of the inflammation mechanism, which subsequently triggers additional cell damage (reperfusion injury). Such a highly potent agent is colchicine - and its derivatives - since exactly this spectrum of action is also triggered.

Particularly, the stent 1 can comprise a scaffold 3, e.g. formed out of a metal alloy, coated with the polymer the matrix 4, the polymer matrix 4 comprising said α-ketoglutaric acid-based polymer 40, wherein preferably a drug, such as colchicine or sirolismus, is dispersed in said polymer matrix 4. The scaffold/stent can be spun around with the α-ketoglutaric acid-based polymer 40 to ensure higher availability of the tissue-protective agent.

According to a further embodiment shown in Fig. 2, said α-ketoglutaric acid-based polymer 40 can be used as a resorbable graft material. Such a graft 1 can be used to replace a vessel section comprising the aneurysm thereby connecting two intact vessel portions 6.

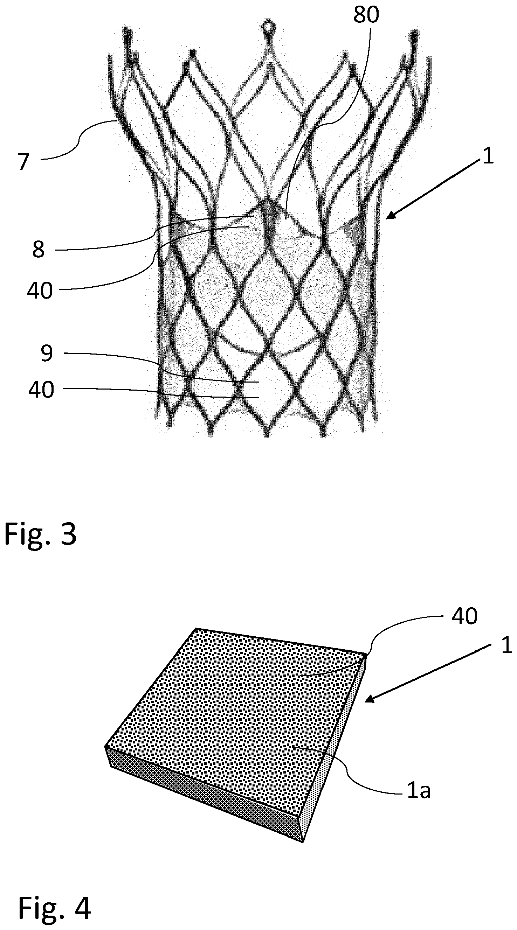

According to yet another embodiment shown in Fig. 3, the medical device 1 can be an implant such as a prosthetic heart valve 1, the heart valve prosthesis 1 comprising a component 8, 9 comprising said α-ketoglutaric acid-based polymer 40. Particularly, this component can be a skirt 9 of the prosthesis 1 mounted to a scaffold 7 of the prosthesis. Alternatively, or in addition, the component 8 can be valve member 8 comprising a plurality of valve leaflets 80. With the component 8 being a plurality of valve leaflets a prosthesis can be provided that comprises a synthetic and resorbable heart valve substrate.

According to a further embodiment shown in Fig. 4, the medical device 1 is a wound patch 1 that can comprise a surface 1a comprising said α-ketoglutaric acid-based polymer 40.

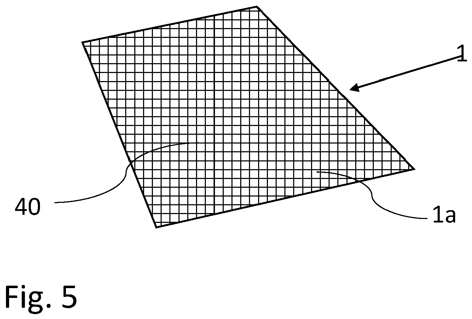

According to yet another embodiment depicted in Fig. 5, the medical device 1 can also be a tissue 1 or surgical mesh 1, for instance for pediatric surgery - that are made from an α-ketoglutaric acid-based polymer 40 or comprise such a polymer 40. Particularly, such a mesh 1 can be configured for the treatment of a hernia. Also, here, the mesh 1 can in particular comprise a surface 1a that comprises said polymer 40 or is made out of said polymer 40.

Furthermore, in embodiments of the invention, the ketone compounds discussed so far, can of course also be combined with an isocyanate to form polymers. To ensure maximum biocompatibility, a reaction with putrescine diisocyanate would also be possible. Particularly in case said α-ketoglutaric acid-based polyesters are used, a pH-controlled hydrolysis can be obtained, which can be further fine-tuned by influencing the hydrophobic properties of the formulation.

The present invention offers the advantages of providing a biocompatible, resorbable material with a beneficial pharmaceutical effect that can be applied in a variety of different medical devices, particularly implants.

## Claims

1. A medical device (1) comprising an α-ketoglutaric acid-based polymer (40).

2. The medical device according to claim 1, wherein the α-ketoglutaric acid-based polymer (40) is an α-ketoglutaric acid-based polyester.

3. The medical device according to claim 1 or 2, wherein α-ketoglutaric acid-based polymer (40) is selected from the group comprised of: α-ketoglutaric hexanediol polyester, α-ketoglutaric isosorbide polyester, and α-ketoglutaric acid-based polyester.

4. The medical device according to one of the preceding claims, wherein the medical device (1) is a stent.

5. The medical device according to claim 4, wherein the stent (1) is a drug eluting stent configured to release a drug.

6. The medical device according to claim 5, wherein the stent comprises a scaffold (3) coated with a polymer matrix (4), the polymer matrix (4) comprising said α-ketoglutaric acid-based polymer (40), the drug being dispersed in said polymer matrix (4).

7. The medical device according to claim 6, wherein said α-ketoglutaric acid-based polymer (40) is spun onto the scaffold (3).

8. The medical device according to one of the claims 5 to 7, wherein the drug is selected from the group comprised of: sirolimus and derivatives thereof, colchicine, paclitaxel and combinations of the aforementioned drugs.

9. The medical device according to one of the claims 1 to 3, wherein the medical device (1) is a graft, particularly a graft for repairing an aneurysm.

10. The medical device according to one of the claims 1 to 3, wherein the medical device (1) is a heart valve prothesis, the heart valve prosthesis comprising a component (8, 9) comprising said α-ketoglutaric acid-based polymer (40).

11. The medical device according to claim 10, wherein said component (8, 9) is one of: a skirt (9); a valve member (8) of the heart valve prosthesis (1), the valve member (8) comprising a plurality of valve leaflets (80).

12. The medical device according to one of the claims 1 to 3, wherein the medical device (1) is a wound patch comprising a surface (1a), the surface (1a) being formed by said α-ketoglutaric acid-based polymer (40) or comprising said α-ketoglutaric acid-based polymer (40).

13. The medical device according to one of the claims 1 to 3, wherein the medical device (1) is a surgical mesh, particularly a hernia mesh, the surgical mesh comprising a surface (1a) being formed by said α-ketoglutaric acid-based polymer (40) or comprising said α-ketoglutaric acid-based polymer (40).

14. A use of an α-ketoglutaric acid-based polymer (40) as a material, particularly a surface material, of a medical device (1), particularly of an implantable medical device.

15. The use according to claim 14, wherein said α-ketoglutaric acid-based polymer (40) is one of: an α-ketoglutaric acid-based polyester, α-ketoglutaric hexanediol polyester, α-ketoglutaric isosorbide polyester.
